## Europäisches Patentamt

## European Patent Office

(11) Publication number: **0 225 686**
**B1**

## Office européen des brevets

(12) **EUROPEAN PATENT SPECIFICATION**

(45) Date of publication of the patent specification:
**18.10.89**

(51) Int. Cl.⁴: **C10G 47/12**
**// B01J23/24, B01J23/40,**
**B01J23/74**

(21) Application number: **86306784.9**

(22) Date of filing: **02.09.86**

(54) Process for hydrotreating residual petroleum oil.

(30) Priority: **10.09.85 US 774520**

(43) Date of publication of application:
**16.06.87 Bulletin 87/25**

(45) Publication of the grant of the patent:
**18.10.89 Bulletin 89/42**

(84) Designated Contracting States:
**AT BE DE FR GB IT NL SE**

(56) References cited:
**GB-A- 1 599 874**
**US-A- 4 033 858**
**US-A- 4 138 326**
**US-A- 4 313 817**

(73) Proprietor: **MOBIL OIL CORPORATION, 150 East 42nd Street, New York New York 10017(US)**

(72) Inventor: **Angevine, Philip Jay, 10 Kent Court, West Deptford New Jersey 08051(US)**
Inventor: **Landis, Michael Eugene, 26 No. Horace Street, Woodbury New Jersey 08096(US)**
Inventor: **Degnan, Thomas Francis, Jr., 40 N. Homestead Drive, Yardley Pennsylvania 19067(US)**

(74) Representative: **Cooper, John Anthony et al, Mobil Court 3 Clements Inn, London WC2A 2EB(GB)**

## Description

This invention relates to a process of upgrading residual petroleum oils by hydrotreating.

Residual petroleum oil fractions such as those heavy fractions produced by atmospheric and vacuum crude distillation columns, are typically characterized as being undesirable as feedstocks for most refining processes due primarily to their high metals, nitrogen, Conradson carbon residue and/or sulfur content. The presence of high concentrations of metals and sulfur and their compounds preclude the effective use of such residua as chargestocks for cracking, hydrocracking and coking operations as well as limiting the extent to which such residua can be used as fuel oil. Perhaps the single most undesirable characteristic of such feedstocks is their high metals content. Principal metal contaminants are nickel and vanadium. Sulfur is also undesirable in a process unit chargestock, since sulfur contributes to corrosion of mechanical equipment and creates difficulties in treating products and flue gases. At typical cracking conversion rates, about one half of the sulfur charged to the unit is converted to $H_2S$ gas which must be removed from the light gas product.

Increasingly, residual oil is being upgraded into lighter petroleum products, notably transportation fuels, by hydrotreating the residual oil. This treatment of residual oil in the presence of a catalyst and hydrogen to transform it into lighter petroleum products is difficult because of the aforementioned presence of sulfur, nickel and vanadium in the molecules of the residual oil.

Catalysts having different size pores have been used to hydrotreat residual oil. For example, it has been recognized that large pore catalysts remove metals faster than small pore catalysts whereas small pore catalysts remove sulfur faster than large pores.

In the prior art, several catalysts, each having individual pore characteristics, have been employed to hydrotreat residual oils. For example, U.S. 4,054,508, teaches a process whereby a resid is passed over a large pore catalyst in order to demetallize the resid which is thereafter passed over a small pore desulfurizing catalyst. U.S. 4 267 071 teaches a hydrotreating catalyst whose pore size distribution is specifically tailored to complement the particular resid feed to be processed.

U.S. 4 389 304 discloses a hydrodesulfurization or hydrodenitrogenation catalyst containing alumina loaded with cobalt, molybdenum and titanium, while U.S. 4 287 050 discloses hydrodesulfurization with alumina promoted with zinc titanate, cobalt and molybdenum.

U.S. 3 865 895 discloses the use of layered complex metal silicates such as chrysotile in processes such as hydrodesulfurization and hydrodenitrogenation. However, the interlayer separation is relatively small for demetallation as is evidenced by d-spacings no greater that about 7.9 angstroms.

Because the principal metal contaminants such as nickel, vanadium, iron and copper are often associated with large planar organometallic complexes such as metalloporphyrins and similar cyclic tetrapyrroles, in the asphaltene fractions of resids, it would be particularly advantageous to employ a hydrotreating catalyst highly selective towards these shapes in resid upgrading. Such a material should exhibit not only hydrogenation activity, but also thermal stability such that the catalyst is capable of withstanding conditions ordinarily encountered during hydrotreating.

It has now been found that thermally stable layered metal compounds containing polymeric oxides between the layers may be employed in hydrotreating catalysts used to upgrade residual oil.

Accordingly the invention resides in a method for hydrotreating residual oil which comprises contacting said oil with a hydrotreating catalyst which contains a catalytic metal and a thermally stable composition comprising a layered metal chalcogenide containing interlayer inorganic polymeric oxide pillars and having a d-spacing of at least 10 Angstrom at hydrotreating conditions which include a temperature of 357°C to 454°C (675°F to 850°F), a hydrogen partial pressure of at least 2860 kPa (400 psig) and a liquid hourly space velocity 0.1 and 10 hr⁻¹.

Preferably, a hydrogenation component comprising at least one metal selected from Group VIB and Group VIII of the Periodic Table is incorporated in the hydrotreating catalyst. The catalyst enhances removal of undesirable metals, sulfur, nitrogen and Conradson carbon residue.

Many layered materials are known which have three-dimensional structures which exhibit their strongest chemical bonding in only two dimensions. In such materials, the stronger chemical bonds are formed in two-dimensional planes and a three-dimensional solid is formed by stacking such planes on top of each other. However, the interactions between the planes are weaker than the chemical bonds holding an individual plane together. The weaker bonds generally arise from interlayer attractions such as Van der Waals forces, electrostatic interactions, and hydrogen bonding. In those situations where the layered structure has electronically neutral sheets interacting with each other solely through Van der Waals forces, a high degree of lubricity is manifested as the planes slide across each other without encountering the energy barriers that arise with strong interlayer bonding. Graphite is an example of such a material. The silicate layers of a number of clay materials are held together by electrostatic attraction between by ions located between the layers. In addition, hydrogen bonding interactions can occur directly between complementary sites on adjacent layers, or by way of interlayer bridging molecules.

Laminated materials such as clays may be modified to increase their surface area. In particular, the interlayer spacing can be increased substantially by absorption of various swelling agents such as water, ethylene glycol, amines and ketones, which enter the interlayer space and push the layers apart. However, the interlayer spaces of such layered materials tend to collapse when the molecules occupying the space are removed, for example, by exposing the clays to high temperatures. Accordingly, such layered materials having enhanced surface area are not suited for use in chemical processes involving even moderately severe conditions.

The extent of interlayer separation can be estimated by using standard techniques such as X-ray diffraction to determine the basal spacing, also known as "repeat distance" or "d-spacing." These values indicate the distance between, for example, the uppermost margin of one layer and the uppermost margin of its adjoining layer. If the layer thickness is known, the interlayer spacing can be determined by subtracting the layer thickness from the basal spacing.

The method of the present invention utilizes a hydrotreating catalyst prepared from a layered starting material which contains anionic ion exchange sites having interlayer or interspathic cations associated therewith. Such cations may include hydrogen ion, hydronium ion and alkali metal cation. The starting material is treated with a "propping" agent, conveniently comprising a source of an organic cation such as organoammonium ion, in order to effect an exchange of or addition to the interlayer cations and thereby separate the layers of the starting material. For example, where the interlayer cations are hydrogen or hydronium ions, the source of organic cation, may include a neutral compound such as organic amine which is converted to a cationic analogue during the "propping" treatment. The foregoing treatment results in the formation of a layered metal oxide of enhanced interlayer separation depending upon the size of the organic cation introduced. In one embodiment, a series of organic cation exchanges is carried out. For example, an organic cation may be exchanged with an organic cation of greater size, thus increasing the interlayer separation in a step-wise fashion. Preferably, contact of the layered material with the propping agent is conducted in aqueous medium so that water is trapped within the interlayer spaces of the propped species.

After the ion exchange, the organic-"propped" species is treated with a compound capable of conversion, preferably by hydrolysis, to a polymeric oxide. The "propped" layered material containing the polymeric oxide precursor is then treated to produce polymeric oxide pillars separating the layers. Where the treatment involves hydrolysis, this may for example be carried out using water already present in organic-"propped" layered material.

It is preferred that the organic cation deposited between the layers is capable of being removed from the layered material without substantial disturbance or removal of the polymeric oxide or oxide precursor. For example, organic cations such as n-octylammonium may be removed by calcination or chemical oxidation, although preferably by calcination and preferably after the polymeric oxide precursor has been converted to the polymeric oxide.

The resulting oxide-pillared product exhibits high surface area, e.g., greater than 200, 400, or even 600 $m^2/g$, and thermal stability.

The layered materials used in producing the catalyst employed in the present invention are layered chalcogenides, preferably oxides, of elements having an atomic number from 13 to 15, 21 to 33, 39 to 51, 57 to 83 or greater than 90. Preferably, the layered oxide is "non-swellable" which is intended to distinguish from conventional clays which contain octahedrally coordinated metal oxide sheets bonded to tetrahedrally coordinated silica sheets and which undergo substantial swelling, sometimes by an essentially unbounded amount, when contacted with water. As used herein in relation to a layered oxide material, the term "non-swellable" is defined as meaning a layered oxide material which, when contacted with at least 10 grams of water per gram of the layered oxide at 23°C for 24 hours, exhibits an increase in d-spacing no greater than 5 Angstrom as compared with the anyhydrous material. Included among these materials are $H_2Ti_3O_7$, $Na_2Ti_3O_7$ and $KTiNbO_5$ as well as certain layered silicates, for example, the metasilicates magadiite, natrosilite, kenyaite, makatite and kanemite. Other suitable starting materials include layered clays, such as bentonite, although these are swellable in water. With certain layered starting materials, for example layered silicates, it has been found to be preferable to treat the silicate with one or more polar solvents prior to or during exchange with the source of organic cation. The polar solvent used should exhibit electric dipole moments in the gas phase of at least 3.0 Debyes (D), preferably at least 3.5 Debyes, most preferably at least about 3.8D. Examples of suitable solvents are water, dimethylsulfoxide (DMSO) and dimethylformamide (DMF). A table of selected organic compounds and their electric dipole moments can be found in CRC Handbook of Chemistry and Physics, 61st Edition, 1980-1981 at pages E-64 to E-66. It is believed that the treatment of the oxide starting material with one or more highly polar solvents facilitates the introduction of the source of organic cation between the layers of the starting material.

In one preferred embodiment, the starting material is a layered oxide of Group IV A metal such as titanium, zirconium and hafnium, with a layered titanate, e.g., a trititanate such as $Na_2Ti_3O_7$, being particularly preferred. Trititanates are commercially available materials whose structure consists of anionic sheets of titanium octahedra with interlayer alkali metal cations. A method for making such material may be found in U.S. Patent 2,496,993.

As previously stated, the starting layered oxide material is treated with an organic compound capable of forming cationic species such as organophosphonium or organoammonium ion, before adding the polymeric oxide source. Insertion of the organic cation between the adjoining layers serves to physically separate the layers in such a way as to make the layered oxide receptive to the interlayer addition of an electrically neutral, hydrolyzable, polymeric oxide precursor. In particular, alkylammonium cations have been found useful in the present invention. Thus $C_3$ and larger alkylammonium, e.g., n-octylammonium, cations are readily incorporated within the interlayer species of the layered oxides, serving to prop open the layers in such a way as to allow incorporation of the polymeric oxide precursor. The extent of the interlayer spacing can be controlled by the size of the organoammonium ion employed so that, with a trititanate as the layered oxide starting material, use of the n-propylammonium cation will achieve a d-spacing of about 10.5A, whereas to achieve a d-spacing of 20A an n-octylammonium cation or a cation of equivalent length is required. Indeed, the size and shape of the organic cation can affect whether or not it can be incorporated within the layered oxide structure at all. For example, bulky cations such as tetrapropylammonium are generally undesirable for use in the present method, with ammonium cations derived from n-alkyl primary amines, more preferably primary monoamines, being preferred. The organic ammonium cations separating the oxide layers may be formed in situ by reaction of the neutral amine species with interlayer hydrogen or hydronium cations of the layer oxide starting material. Alternatively where the interlayer cations of the layered oxide starting material are alkali metal cations, the organic ammonium cation may be formed by initially combining an amine and an aqueous acid solution, such as hydrochloric acid, and then treating the layered oxide with the resulting aqueous organoammonium ion solution. In either case, the treatment is conducted in aqueous media so that water is then available to hydrolyze the electrically neutral, hydrolyzable polymeric oxide precursor subsequently introduced into the "propped" product.

The polymeric oxide pillars formed between the layers of the oxide starting material may include an oxide of zirconium or titanium or more preferably of any element selected from Croup IVB of the Periodic Table (Fischer Scientific Company Cat. No. 5-702-10), other than carbon, and most preferably include polymeric silica.

The polymeric oxide pillars are formed from a precursor material which is preferably introduced between the layers of the organic propped species as a cationic, or more preferably electrically neutral, hydrolyzable compound of the desired Group IVB elements.

The precursor material is preferably an organometallic compound which is a liquid under ambient conditions. Suitable polymeric silica precursor materials include tetrapropylorthosilicate, tetramethylorthosilicate and, most preferably, tetraethylorthosilicate.

After hydrolysis to produce the polymeric oxide pillars and calcination to remove the organic propping agent, the final pillared product may contain residual exchangeable cations. For example, sodium titanate pillared with polymeric silica may contain 2-3% of weight of residual sodium. Such residual cations can be ion exchanged by methods well known with other cationic species to provide or alter the catalytic activity of the pillared product. Suitable replacement cations include cesium, cobalt, nickel, copper, zinc, manganese, platinum, lanthanum, aluminum and mixtures thereof.

In the preferred embodiment, where the layer oxide is a titanate and the pillars are formed of silica, the resulting silicotitanate product exhibits the characteristic X-ray diffraction pattern shown in Table 1 below.

Table 1

Composite list of principal X-ray powder*
Diffraction peaks for silicotitanates

| Line Number | (2 Theta–2 Theta) (Minimum Maximum) | 100 $I/I_0$ (Relative Intensity) Range |
|---|---|---|
| 1 | less than or equal to 8.7 | VS to W |
| 2 | 11.1–14.3 | S to W |
| 3 | 11.8–15.2 | M to W |
| 4 | 24.5–25.0 | VS to W |
| 5 | 25.0–25.4 | M to W |
| 6 | 28.5–30.2 | VS to W |
| 7 | 29.8–30.6 | S to W |
| 8 | 33.0–33.5 | S to W |
| 9 | 43.2–43.5 | M to W |
| 10 | 44.2–44.7 | M to W |
| 11 | 48.5–48.9 | VS to M |
| 12 | 52.7–52.9 | W |

* 2 Theta minimum – 2 Theta maximum = Range of 2 Theta-values observed for eight specific pillared silicotitanates

These values were determined by standard techniques. The radiation was the K-alpha doublet of copper, and a scintillation counter spectrometer was used. The peak heights, I, and the positions as a function of 2 times theta, where theta is the Bragg angle, were determined. From these, the relative intensities, $I/I_0$ where $I_0$ is the intensity of the strongest line or peak, and d is the interplanar spacing in angstroms (A), corresponding to the recorded lines, were calculated. The relative intensity in the table above is expressed as follows:

| Relative Intensity | 100 $I/I_0$ |
|---|---|
| VS (Very Strong) | 60–100 |
| S (Strong) | 40–60 |
| M (Medium) | 20–40 |
| W (Weak) | 0–20 |

Variations in the interplanar spacing and relative intensity may occur as a result of ion exchange, changes in the composition of the silicotitanate, or exposure to calcination conditions:

When used as a catalyst, in the present hydrotreating method, the layered and pillared oxides described above preferably contain a Group VIB, e.g. chromium, molybdenum or tungsten, and/or a Group VIII metal, e.g. platinum, which can be introduced into the catalyst by impregnation or, where the pillared material has ion exchange capability, by ion exchange.

Layered metal oxides containing an interspathic polymeric oxide pillars such as those described above have now been found to be useful in upgrading and particularly in demetallation of residual oil by hydrotreating. The layered materials useful in the method of the present invention preferably exhibit significant interlayer separation and possess a d-spacing of at least about 10 angstroms. The polymeric oxide pillars serve to maintain this interlayer separation even at the conditions of temperature and pressure encountered during resid upgrading. The layered metal oxides described above are particularly useful as resid upgrading catalysts because their layered structure results in pores which are relatively long and narrow. Such pore shapes are believed to readily accomodate all or part of the large planar metal complexes found in asphaltene portions of resid and thus to permit access of such metal complexes to catalytic sites within the catalysts.

The present method is suitable for hydrotreating 340°C+ and preferably 540°C+ residual oil and involves contacting the residual oil with hydrogen in the presence of the above-described layered oxide hydrotreating catalyst, which preferably contains a metal selected from Group VIB and Group VIII metals. Preferably, the hydrotreating catalyst comprises a layered silicotitanate. The metals removed from the residual oil may include such common crude oil metal contaminants as nickel, vanadium, iron, copper, zinc and sodium, which are often in the form of large organometallic complexes such as metal porphyrins or asphaltenes.

The present method may be carried out by contacting the metal and/or sulfur contaminated feedstock with the above-described hydrotreating catalyst under hydrogen pressures of at least 2860 kPa (400 psig), temperatures ranging between 357 to 454°C (675 to 850°F) and liquid hourly space velocities between 0.1 and 10. Preferably these conditions include hydrogen pressures between 7000 to 17000 kPa (1000 to 2500 psig), temperatures between 370 to 440°C (700 to 825°F), and liquid hourly space velocities between 0.2 and 1.0 hr⁻¹.

The catalytic hydrotreating may take place in any suitable hydrotreating reactor, preferably a fixed bed downflow (trickle bed) reactor. Other suitable hydrotreaters include moving bed downflow ("bunker") reactors, fluidized bed or ebullated bed reactors and fixed bed upflow reactors.

The use of the present hydrotreating catalysts in resid upgrading is particularly desirable because they exhibit greater activity, particularly in metals removal. Accordingly a refiner can attain the required degree of metals removal with less catalyst in a smaller reactor. In view of the high pressures required for hydroprocessing resids, it is highly desirable from an economic standpoint to utilize a reactor of the smallest size allowable. Alternatively, hydrotreating with the catalysts of the present method in larger reactors allows a refiner to operate at lower reaction severities or to attain hydrotreated resids of improved quality. In view of this, the method of the present invention is believed particularly useful in pretreating FCC feed or producing metallurgical grade coke.

The following examples are given to further describe the present invention.

Example 1 (Comparative)

A support was prepared by extruding a mixture of water and alpha alumina monohydrate ("Catapal SB") to 0.8mm (1/32") extrudate, drying at about 140°C, and calcining to a distinct pore size distribution. The calcined extrudate was then sequentially impregnated via the conventional incipient wetness technique with ammonium heptamolybdate and cobalt chloride. Drying at about 140°C and calcination at 540°C followed each of the two impregnations. The properties of the resultant Catalyst A are shown in Table 2.

Table 2

| CoMo/Al₂O₃ resid demetalation catalyst properties | |
| --- | --- |
| Metals loading (wt %) | |
| CoO | 3.5 |
| MoO₃ | 10.0 |
| Physical properties | |
| Surface Area, m²/g | 109 |
| Real Density, g/cc | 3.629 |
| Particle Density, g/cc | 1.221 |
| Pore Volume, cc/g | 0.543 |
| Avg. Pore Dia., Ang. | 199 |
| Pore size distribution, % pore volume | |
| 0–30 Ang. Diameter | 11 |
| 30–50 | – |
| 50–80 | – |
| 80–100 | 2 |
| 100–150 | 24 |
| 150–200 | 34 |
| 200–300 | 17 |
| 300+ | 12 |

Example 2 (Comparative)

A Mo/Na₂Ti₃O₇ material was prepared by the conventional incipient wetness technique on sodium titanate (Na₂Ti₃O₇) using ammonium heptamolybdate. Following impregnation to 10 wt% MoO₃, the material was calcined in air at 427°C (800°F) for 3 hours. The resultant material was identified as Catalyst B.

Example 3 (Comparative)

Acid titanate, $H_2Ti_3O_7$, was prepared by exchange of Na in $Na_2Ti_3O_7$ with 1 $\underline{M}$ HCl as described: 780.7g 37.4% HCl was diluted to 8 liters total volume with water in a 12 liter 4-necked round bottom flask equipped with a mechanical stirrer, reflux condenser, and thermometer. 500 Grams of $Na_2Ti_3O_7$ were added, and the resulting mixture was heated with stirring at 75-80°C for 24 hours. The solution was then filtered and washed with 2 liters of hot water. This procedure was repeated two further times. After the third exchange, the product was washed with hot water until chloride free. The product after drying in vacuo at 77°C had an X-ray diffraction pattern similar to that reported for $H_2Ti_3O_7$ by H. Izawa, S. Kikkaw, and M. Koizumi, J. Phys. Chem., 86, 5023 (1982) and the following composition (wt%).
$TiO_2$, 93.4
Na, 0.28

Using the resultant acid titanate, a Mo impregnation and calcination similar to Example 2 were completed. The finished material was identified as Catalyst C.

Example 4

Concentrated HCl (315.6g of 37.2% HCl) was dissolved in 700g water, and the resulting solution was stirred and cooled in an ice bath. n-Octylamine (427.1g) was added portionwise, keeping the solution below 50°C. 100 grams of $Na_2Ti_3O_7$ was added, and the mixture was transferred to a 2 liter polypropylene jar and heated at 100°C with occasional stirring for 24 hours. The mixture was filtered, washed with 5 liters hot water, and dried at room temperature for 24 hours. A portion of this dried product (90g) was stirred in 600g tetraethylosthiosilicate for 72 hours at room temperature, filtered, and dried at room temperature for 24 hours. This product was calcined at 540°C (1000°F) in nitrogen for one hour and in air for 2 hours. The properties of this silicotitanate product are provided in Table 3.

Table 3

| Properties of catalyst a silicotitanate of example 4 | |
| --- | --- |
| $SiO_2/TiO_2$, molar | 0.52 |
| Na, wt% | 2.80 |
| Ash, wt% | 98.45 |
| Surface Area, $m^2/g$ | 225.00 |
| Sorption, wt% | |
| $H_2O$ | 10.6 |
| Cyclohexane | 7.6 |
| n-Hexane | 6.1 |

Using the resultant silicotitanate, a Mo impregnation and calcination similar to Example 2 were completed. The finished material was identified as Catalyst D.
Properties of Catalysts B, C, and D are set out in Table 4.

Table 4

| Properties of molybdenum-containing titanate catalysts | | | |
| --- | --- | --- | --- |
| | Catalyst B | Catalyst C | Catalyst D |
| Form | Mo-Impregnated $Na_2Ti_3O_7$ 8/20 Mesh Particles | Mo-Impregnated $H_2Ti_3O_7$ 8/20 Mesh Particles | Mo-Impregnated Silicotitanate 8/20 Mesh Particles |
| Density, g/cc | | | |
| Packed | 0.57 | 0.54 | 0.59 |
| Pore Volume cc/g | NA | NA | 0.497 |
| Surface Area $M^2/g$ | 5 | 5 | 45 |
| Avg. Pore Diameter, Ang. | NA | NA | 44 |

| % Pore volume in pores of varying diameters (angstroms) | |
| --- | --- |
| 0–30 Ang. Diameter | |
| 30–50 Ang. Diameter | 1 |
| 50–80 Ang. Diameter | 1 |
| 80–100 Ang. Diameter | 2 |
| 100–150 Ang. Diameter | 3 |
| 150–200 Ang. Diameter | 2 |
| 200–300 Ang. Diameter | 3 |
| 300+ Ang. Diameter | 87 |

## Example 5

Catalysts A to D, all crushed and sized to a mean particle diameter of 14 to 20 Mesh (Tyler), were employed in hydrotreating an Arabian Light vacuum resid (540°C+ boiling range) described in Table 5 in a shaker bomb apparatus (see J.W. Payne, C.W. Streed and E.R. Kent, "The Shaker Bomb - A New Laboratory Tool for Studying Thermal Processes", Ind. Eng. Chem., 50 (1), 47 (1958)) which approximates resid upgrading activities observed in continuous downflow units.

| Table 5 | |
| --- | --- |
| Arabian light vacuum resid feedstock | |
| Elemental analysis (wt %) | |
| Hydrogen | 10.68 |
| Sulfur | 3.93 |
| Nitrogen | 0.31 |
| CCR | 16.96 |
| Asphaltenes (n–$C_5$) | 10.93 |
| Metals analysis (ppm) | |
| Nickel | 16 |
| Vanadium | 65 |
| Iron | 12 |
| Sodium | 6 |
| Kinematic viscosity (cs) | |
| 212°F (100°C) | 496.2 |
| 300°F (149°C) | 24.6 |

In a series of runs, the catalysts were contact with the resid at the following conditions:

| | |
| --- | --- |
| Oil: catalyst (wt:wt) | 20 |
| Temperature, °C | 400 |
| $H_2$ Pressure, kPa | 13,890 |
| Reaction Time, min | 80 |

At the conclusion of each run, the catalyst and the oil were separated and both components were analyzed. The effectiveness of each titanate catalyst for resid upgrading was determined by comparing the degree of demetalation, desulfurization, CCR removal, and denitrogenation to that observed in an identical run in which a conventional $CoMo/Al_2O_3$ catalyst was used. Thermal contributions were determined from a "blank" run at identical conditions but with no catalyst present.

Table 6 presents the results of this catalyst activity comparison and shows that the molybdenum-impregnated silicotitanate catalyst was superior to the analogously treated sodium titanate and hydrogen titanate, catalysts with respect to CCR, vanadium, and asphaltenes removal. Sulfur removal by all three titanate catalysts was minimal. This was expected since unpromoted $MoO_3$ is known to have little desulfurization activity. Nickel levels in the sodium and hydrogen titanate treated products actually increased due to contamination that can be traced to the stainless steel walls of the shaker bomb. The demetalation activity of the silicotitanate was sufficient to take up this additional nickel.

**Table 6**

Comparison of resid upgrading catalyst performance

| | Thermal | Catalyst A (CoMo/Al) | Catalyst B (Mo/Sodium Titanate) | Catalyst C (Mo/Hydrogen Titanate) | Catalyst D (Mo/Silico Titanate) |
|---|---|---|---|---|---|
| _Conditions_ | | | | | |
| Temp, °C | 400 | | | | |
| Pressure, kPa | 13,890 | | | | |
| Oil/Cat (wt %) | Infinity | 20 | | | |
| Time, min | 80 | | | | |
| _Conversion_ | | | | | |
| to 540°C-, wt% | 13.0 | 32.7 | 4.0 | 17.0 | 24.0 |
| _Total liquid product analysis, wt%_ | | | | | |
| Hydrogen | 10.58 | 10.88 | 10.24 | 10.26 | 10.22 |
| Sulfur | 3.47 | 2.52 | 3.96 | 3.87 | 3.87 |
| Nitrogen | 0.32 | 0.26 | 0.26 | 0.26 | 0.26 |
| Vanadium, ppm | 70 | 33 | 62 | 62 | 24 |
| Nickel, ppm | 16 | 10 | 18 | 19 | 12 |
| CCR, wt% | 16.00 | 14.44 | 16.46 | 16.04 | 16.37 |
| Asphaltenes wt% | 8.52 | 5.40 | 6.99 | 9.76 | 5.56 |
| _Removal, %_ | | | | | |
| Vanadium | 0 | 49 | 5 | 5 | 63 |
| CCR | 6 | 15 | 3 | 6 | 3 |
| Sulfur | 12 | 36 | 0 | 2 | 2 |
| Asphaltenes | 22 | 51 | 36 | 11 | 49 |

Compared to the conventional $CoMo/Al_2O_3$ catalyst, Mo/silicotitanate had greater demetalation activity (63 percent vs. 49 percent). Asphaltene removal activities of the two catalysts were virtually equivalent (49 percent vs 51 percent).

**Claims**

1. A method for hydrotreating residual oil which comprises contacting said oil with a hydrotreating catalyst which contains a catalytic metal and a thermally stable composition comprising a layered metal chalcogenide containing interlayer inorganic polymeric oxide pillars and having a d-spacing of at least 10 Angstrom at hydrotreating conditions which include a temperature ranging from 357°C to 454°C (675°F to 850°F), a hydrogen partial pressure of at least 2860 kPa (400 psig) and a liquid hourly space velocity ranging between 0.1 and 10 hr⁻¹.

2. A method as claimed in claim 1 where in the layered chalcogenide is a metal oxide.

3. A method as claimed in claim 1 or claim 2 wherein the polymeric oxide pillars contain polymeric silica.

4. A method as claimed in any preceding claim wherein the layered compound is a titanate.

5. The method of any preceding claim wherein said catalytic metal includes a Group VIB metal selected from the group consisting of chromium, molybdenum and tungsten.

6. The method of any preceding claim wherein said catalytic metal includes a Group VIII metal.

7. The method of claim 6 wherein said Group VIII metal is selected from iron, cobalt, nickel, palladium and platinum.

8. The method of any preceding wherein said hydrotreating conditions include a temperature ranging from 370°C to 440°C (700°F to 825°F), a hydrogen partial pressure ranging from 7000 to 17,000 kPa (1000 to 2500 psig), and a liquid hourly space velocity ranging between 0.2 to 1.0.

**Patentansprüche**

1. Verfahren zur Hydrobehandlung von Rückstandsöl, bei dem dieses Öl mit einem Hydrobehandlungskatalysator, der ein katalytisches Metall und eine thermisch stabile Zusammensetzung umfaßt, die ein geschichtetes Metallchalcogenid umfaßt, das Zwischenschichtstützen aus anorganischem polymerem Oxid enthält und einen d-Abstand von mindestens 10 Angström aufweist, bei Hydrobehandlungsbedingungen in Kontakt gebracht wird, die eine Temperatur im Bereich von 357°C bis 454°C (675°F bis 850°F), einen Wasserstoffpartialdruck von mindestens 2860 kPa (400 psig) und eine stündliche Flüssigkeits-Raum-Geschwindigkeit im Bereich von zwischen 0,1 und 10 h⁻¹ umfassen.

2. Verfahren nach Anspruch 1, worin das geschichtete Chalkogenid ein Metalloxid ist.

3. Verfahren nach Anspruch 1 oder 2, worin die polymeren Oxidstützen polymeres Siliciumdioxid enthalten.

4. Verfahren nach einem der vorstehenden Ansprüche, worin die geschichtete Verbindung Titanat ist.

5. Verfahren nach einem der vorstehenden Ansprüche, worin das katalytische Metall ein Metall der Gruppe VIB umfaßt, das aus der Gruppe ausgewählt ist, die aus Chrom, Molybdän und Wolfram besteht.

6. Verfahren nach einem der vorstehenden Ansprüche, worin das katalytische Metall ein Metall der Gruppe VIII umfaßt.

7. Verfahren nach Anspruch 6, worin das Metall der Gruppe VIII aus Eisen, Kobalt, Nickel, Palladium und Platin ausgewählt ist.

8. Verfahren nach einem der vorstehenden Ansprüche, worin die Hydrobehandlungsbedingungen eine Temperatur im Bereich von 370°C bis 440°C (700°F bis 825°F), einen Wasserstoffpartialdruck im Bereich von 7000 bis 17 000 kPa (1000 bis 2500 psig) und eine stündliche Flüssigkeits-Raum-Geschwindigkeit im Bereich zwischen 0,2 bis 1,0 umfassen.

**Revendications**

1. Procédé pour l'hydrotraitement d'une huile résiduelle, qui consiste à mettre en contact ladite huile avec un catalyseur d'hydrotraitement qui contient un métal catalytique et une composition thermiquement stable comprenant un chalcogénure métallique feuilleté contenant des piliers d'oxydes polymères inorganiques entre les feuillets et ayant une distance d d'au moins 10 angstroms, dans des conditions d'hydrotraitement comprenant une température comprise entre 357 et 454°C (entre 675 et 850°F), une pression partielle d'hydrogène d'au moins 2860 kPa (400 psig) et une vitesse spatiale liquide horaire comprise en- tre 0,1 et 10 h⁻¹.

2. Procédé selon la revendication 1, dans lequel le chalcogénure feuilleté est un oxyde métallique.

3. Procédé selon la revendication 1 ou la revendication 2, dans lequel les piliers d'oxyde polymère contiennent de la silice polymère.

4. Procédé selon l'une quelconque des revendications précédentes, dans lequel le composé feuilleté est un titanate.

5. Procédé selon l'une quelconque des revendications précédentes, dans lequel ledit métal catalytique comprend un métal du Groupe VIB choisi dans le groupe comprenant le chrome, le molybdène et le tungstène.

6. Procédé selon l'une quelconque des revendications précédentes, dans lequel ledit métal catalytique comprend un métal du Groupe VIII.

7. Procédé selon la revendication 6, dans lequel ledit métal du Groupe VIII est choisi dans le groupe comprenant le fer, le cobalt, le nickel, le palladium et le platine.

8. Procédé selon l'une quelconque des revendications précédentes, dans lequel lesdites conditions d'hydrotraitement comprennent une température comprise entre 370 et 440°C (entre 700 et 825°F), une pression partielle d'hydrogène comprise entre 7000 et 17 000 kPa (entre 1000 et 2500 psig) et une vites- se spatiale liquide horaire comprise entre 0,2 et 1,0.